# EUROPÄISCHE PATENTSCHRIFT

(11) **EP 1 272 150 B1**
(45) Veröffentlichungstag und Bekanntmachung des Hinweises auf die Patenterteilung: **01.03.2006**
(21) Anmeldenummer: 01943212.9
(22) Anmeldetag: 31.03.2001
(51) Int. Cl.: A61Q 5/10, A61K 8/49

(54) **FÄRBEMITTEL MIT RUTHENIUMVERBINDUNGEN**
COLORING AGENTS CONTAINING RUTHENIUM COMPOUNDS
COLORANTS CONTENANT DES COMPOSES DE RUTHENIUM

(30) Priorität: 12.04.2000 DE 10018160
(43) Veröffentlichungstag der Anmeldung: 08.01.2003
(73) Patentinhaber: Henkel Kommanditgesellschaft auf Aktien, 40589 Düsseldorf (DE)
(72) Erfinder: MAYER, Bernd, 40593 Düsseldorf (DE); HELLER, Melita, 40591 Düsseldorf (DE); HÖFFKES, Horst, 40595 Düsseldorf (DE)
(86) Internationale Anmeldenummer: PCT/EP2001/003693
(87) Internationale Veröffentlichungsnummer: WO 2001/078665

(56) Entgegenhaltungen:
- WO-A-01/12143
- WO-A-98/01106
- DE-A- 19 852 972
- DE-A- 19 859 681
- DE-A- 19 937 311
- US-A- 3 429 646
- DATABASE WPI Section Ch, Week 199015 Derwent Publications Ltd., London, GB; Class A87, AN 1990-111382 XP002175885 & JP 02 061182 A (AGENCY OF IND SCI & TECHNOLOGY), 1. März 1990 (1990-03-01)

## Beschreibung

Die vorliegende Erfindung betrifft ein Verfahren zum Färben keratinischer Fasern, bei dem spezielle Verbindungen des Rutheniums als katalytisch wirksame Aktivatoren eingesetzt werden sowie entsprechende Mittel zum Färben der Fasern.

Für das Färben von keratinhaltigen Fasern, z.B. Wolle, Pelzen und insbesondere menschlichen Haaren, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung.

Direktziehende Farbstoffe werden unter schonenden Bedingungen appliziert. Ihr Nachteil liegt jedoch darin, daß die Färbungen häufig nur über unzureichende Echtheitseigenschaften verfügen. Mit Oxidationsfarbstoffen lassen sich zwar intensive Färbungen mit guten Echtheitseigenschaften erzielen, die Entwicklung der Farbe geschieht jedoch in der Regel unter dem Einfluß von H₂O₂ oder H₂O₂-Addukten, was Schädigungen der Faser zur Folge haben kann.

Auch Oxidationsfärbemittel mit Luftsauerstoff als sehr mildem Oxidationsmittel wurden bereits vorgeschlagen; im allgemeinen laufen die Oxidationen mit Luftsauerstoff jedoch nicht vollständig ab. Insbesondere in Oxidationshaarfärbemitteln, die in der Regel in einem cremeartigen kosmetischen Träger appliziert werden, findet keine zufriedenstellende Diffusion des Luftsauerstoff zu den Farbstoffvorprodukten statt. Weiterhin wurde, beispielsweise in der EP-B1-0 548 620, vorgeschlagen, den Gehalt an Wasserstoffperoxid in den Oxidationsfärbemitteln auf 1 Gew.-% und weniger zu begrenzen, dem Mittel aber gleichzeitig zur Aktivierung spezielle Enzyme, Peroxidasen, zuzufügen. Diese Ansätze haben aber bisher nicht vollständig überzeugt.

Es besteht daher nach wie vor die Aufgabe, Oxidationsfärbemittel zu entwickeln, die aufgrund ihres geringeren Gehaltes an Oxidationsmitteln schonendere Färbungen ermöglichen, ohne das Färbeergebnis negativ zu beeinflussen. Weiterhin ist es in hohem Maße wünschenswert, den pH-Wert der Färbemittel auf einen Wert um den Neutralpunkt zu senken, um möglichen Schädigungen der Faser aufgrund der üblicherweise starken Alkalinität der Färbemittel vorzubeugen.

Es wurde nunmehr überraschenderweise gefunden, daß durch Verwendung spezieller Verbindungen des Rutheniums als Oxidationskatalysatoren zur Färbung keratinischer Fasern
- die Menge des Oxidationsmittels stark reduziert werden kann und/oder
- die Ausfärbung im neutralen pH-Bereich erfolgen kann.

Der Einsatz von Übergangsmetallkomplexen im Zusammenhang mit einer oxidativen Haarfärbung ist prinzipiell bekannt. So wurde in der europäischen Patentanmeldung EP-A2-507 448 vorgeschlagen, die Haare vor dem eigentlichen Färbeprozeß mit einem Übergangsmetallkomplex, enthaltend die Liganden 2,2'-Dipyridyl oder o-Phenanthrolin, zu behandeln, um das Haar gegen einen oxidativen Abbau des Cysteins zu stabilisieren. In der US3429646 werden Haarfärbeverfahren offenbart, bei denen Übergangsmetallkomplexe, beispielsweise von Ruthenium, mit cyclischen und acyclischen Liganden zur direkten Färbung von Haaren eingesetzt werden. In der deutschen Patentschrift DE-C1-195 34 214 wurde weiterhin vorgeschlagen, Kupferkomplexe als Oxidationskatalysatoren in Haarfärbemitteln einzusetzen, die als Entwicklerkomponenten Derivate des 4-(2,5-Diaminophenoxymethyl)-1,3-dioxolans enthalten. Schließlich wurde in der deutschen Anmeldung DE-A1-197 57 510 vorgeschlagen, Übergangsmetallkomplexe mit Liganden vom Salen-Typ als Oxidationskatalysatoren einzusetzen. Dennoch bestand weiterhin Bedarf an neuartigen Oxidationskatalysatoren für die oxidative Haarfärbung.

Ein erster Gegenstand der vorliegenden Erfindung ist daher ein Verfahren zum Färben keratinischer Fasern, insbesondere menschlicher Haare, bei dem eine Zubereitung, enthaltend (A) mindestens ein Farbstoffvorprodukt und (B) mindestens ein einfaches Rutheniumsalz und/oder einen Rutheniumkomplex mit acyclischen Liganden, auf die Fasern aufgetragen wird und nach einer Einwirkzeit wieder abgespült wird, mit der Maßgabe, daß die Rutheniumsalze und/oder Rutheniumkomplexe nicht in Hohlräumen von Käfigverbindungen eingeschlossen sind.

Die erfindungsgemäßen Rutheniumsalze und/oder Rutheniumkomplexe mit acyclischen Liganden werden im Folgenden als Rutheniumverbindungen bezeichnet. Die Rutheniumkomplexe enthalten erfindungsgemäß ausschließlich acyclische Liganden.

Erfindungsgemäß sind unter acyclischen Liganden sämtliche Liganden zu verstehen, die kein Ringsystem enthalten.

Mithilfe der erfindungsgemäßen Rutheniumverbindungen lassen sich überraschenderweise intensivere Färbungen bei gleicher Metallkonzentration als bei Katalysatoren des Standes der Technik erzielen.

Erfindungsgemäß besteht hinsichtlich der Oxidationsstufe des Ruthenium keinerlei Einschränkung, als geeignet haben sich Rutheniumverbindungen erwiesen, die das Ruthenium in Oxidationsstufen zwischen -2 und +7 enthalten. In einer bevorzugten Ausführungsform werden Verbindungen mit Rutheniumkationen der Oxidationsstufe +3 eingesetzt.

Als Anionenliganden beziehungsweise als Gegenionen kommen insbesondere die Halogenide, wie Fluorid, Chlorid, Bromid und Iodid, Nitrat, Cyanid, Actetat, Trifluoracetat, Formiat, Hydroxid, Sulfat, Phosphat, Chlorat, Perchlorat, Carbonat, Citrat, sowie komplexe Anionen, wie Hexafluorophosphat und Tetrafluoroborat, in Frage. Neben diesen können aber auch mehrzähnige anionische Liganden in den erfindungsgemäßen Rutheniumverbindungen enthalten sein, wie beispielsweise die Salze der Ethylendiamintetraessigsäure oder der Nitrilotriessigsäure und die (NO₂)⁻-Gruppe. Unter einer (NO₂)⁻-Gruppe soll im Rahmen dieser Anmeldung sowohl ein Nitro-Ligand, der über das Stickstoffatom an das Rutheniumkation gebunden ist, als auch ein Nitrito-Ligand, der über ein Sauerstoffatom an das Rutheniumkation gebunden ist, verstanden werden. Die (NO₂)⁻-Gruppe kann an ein Rutheniumkation auch chelatbildend gebunden sein oder sie kann zwei Rutheniumkationen asymmetrisch oder µ¹-O-verbrücken.

Erfindungsgemäß bevorzugt sind Rutheniumverbindungen, die mindestens ein Rutheniumkationen und mindestens ein Chloridanion enthalten. Erfindungsgemäß besonders bevorzugte Rutheniumverbindungen enthalten mindestens ein Rutheniumkation und zum Ladungsausgleich eine entsprechende Anzahl Chloridanionen.

Neben den Gegenionen können die Rutheniumverbindungen noch weitere neutrale, acyclische Liganden tragen. Zu den anorganischen Neutralliganden gehören insbesondere Ammoniak und Wasser. Aber auch organische acyclische Neutralliganden, wie Ethylendiamin sowie Phosphine der Formel PR¹R²R³ oder Amine der Formel NR¹R²R³, wobei R¹, R² und R³ stehen jeweils unabhängig voneinander für Wasserstoff oder eine gegebenenfalls substituierte C₁-C₂₀-Alkylgruppe, mit der Maßgabe, daß mindestens einer der Reste R¹, R² und R³ verschieden von Wasserstoff ist, sind erfindungsgemäß umfaßt. Bevorzugt sind insbesondere neutrale, einzähnige Liganden. Ganz besonders bevorzugte Neutralliganden sind Wasser und Ammoniak.

Erfindungsgemäß als besonders geeignet haben sich die Rutheniumverbindungen RuCl₃ x 3 H₂O und [Ru(NH₃)₆]Cl₃ erwiesen.

Die Anwendungszubereitung enthält die Rutheniumverbindungen bevorzugt in einer Menge von 0,0001 bis 1,0 Gew.-%, berechnet als Masse des Ruthenium, bezogen auf die Masse der Gesamtzubereitung.

Die erfindungsgemäßen Rutheniumverbindungen können sowohl in löslicher Form eingesetzt werden, als auch, wenn es sich um kationische Verbindungen handelt, auf einem Träger fixiert sein.

Erfindungsgemäß einsetzbare Trägermaterialien sind beispielsweise Zeolithe, Schichtsilicate oder Sol/Gel-Systeme.

Zeolithe sind Alumosilicate aus dreidimensional vernetzten Aluminat- und Silicat-Tetraederbausteinen. Alle Zeolithe weisen ein ein- oder mehrdimensionales Kanal- und Porensystem auf. Zu den bevorzugten Trägermaterialien gehören die Zeolithe vom Typ A, K, L, P-L, O, T, X, Y und Ω sowie deren Mischungen. Die erfindungsgemäßen Rutheniumverbindungen können sowohl auf der Oberfläche des Zeolithen vorliegen als auch sich innerhalb der Poren befinden. Explizit ausgeschlossen sind dabei erfindungsgemäß Rutheniumverbindungen, die in Zeolithen eingeschlossen sind. Der Ladungsausgleich kann dabei auch zumindest anteilsweise durch den Zeolithen erfolgen.

Erfindungsgemäß einsetzbare Schichtsilicate sind ebenfalls Alumosilicate, die aber nicht die zeolith-typische Porenstruktur aufweisen. Schichtsilicate bilden im suspendierten Zustand vielmehr plättchenförmige Gebilde, auf deren Oberfläche sich die erfindungsgemäßen Rutheniumverbindungen anlagern können. Beispiele für erfindungsgemäße Schichtsilicate ist neben Bentonit, als üblichem Gemisch von Montmorillonit und Kaolinit, weiterhin das unter dem Handelsnamen Dehydril® HT von der Firma Henkel vertriebene Produkt aus äußerst quellfähigem Hectorit.

Erfindungsgemäße Sol/Gel-Systeme sind amorphe, glasartige Substanzen, die aus interpenetrierenden anorganischen (silicatischen) und gegebenenfalls organischen (polymeranalogen) Netzwerken aufgebaut sind. Sol/Gel-Systeme können sowohl auf Basis von Siliciumdioxid als auch auf der Basis von Titandioxid synthetisiert werden.

In einer ersten Ausführungsform der vorliegenden Erfindung kann das Farbstoffvorprodukt (A) ein Oxidationsfarbstoffvorprodukt vom Typ Entwickler sein. Es können auch mehrere Entwickler gemeinsam in den erfindungsgemäßen Mitteln eingesetzt werden.

Entwicklersubstanzen sind üblicherweise aromatische oder heterocyclische Ringsysteme, die durch zwei in ortho- oder para-Stellung zueinander stehende reaktive Gruppen, i.a. Hydroxy- oder Aminogruppen, gekennzeichnet sind. Solche Verbindungen sind beispielsweise primäre aromatische Amine mit einer weiteren, in para- oder ortho-Position befindlichen freien oder substituierten Hydroxy- oder Aminogruppe, ferner Diaminopyridinderivate, heterocyclische Hydrazonderivate oder 4-Aminopyrazolonderivate.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (I) 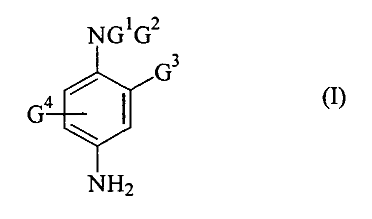
wobei
- G¹ steht für ein Wasserstoffatom, ein C₁- bis C₄-Alkylradikal, ein C₁- bis C₄₋Monohydroxyalkylradikal, ein C₂- bis C₄-Polyhydroxyalkylradikal, ein (C₁- bis C₄)-Alkoxy-(C₁- bis C₄)-alkylradikal, ein 4'-Aminophenylradikal oder ein C₁- bis C₄₋Alkylradikal, das mit einer stickstoffhaltigen Gruppe, einem Phenyl- oder einem 4'-Aminophenylrest substituiert ist;
- G² steht für ein Wasserstoffatom, ein C₁- bis C₄-Alkylradikal, ein C₁- bis C₄₋Monohydroxyalkylradikal, ein C₂- bis C₄-Polyhydroxyalkylradikal, ein (C₁- bis C₄)-Alkoxy-(C₁- bis C₄)-alkylradikal oder ein C₁- bis C₄-Alkylradikal, das mit einer stickstoffhaltigen Gruppe substituiert ist;
- G³ steht für ein Wasserstoffatom, ein Halogenatom, wie ein Chlor-, Brom, Jod- oder Fluoratom, ein C₁- bis C₄-Alkylradikal, ein C₁- bis C₄-Monohydroxyalkylradikal, ein C₁- bis C₄-Hydroxyalkoxyradikal, ein C₁- bis C₄-Acetylaminoalkoxyradikal, ein C₁- bis C₄- Mesylaminoalkoxyradikal oder ein C₁- bis C₄-Carbamoylaminoalkoxyradikal;
- G⁴ steht für ein Wasserstoffatom, ein Halogenatom oder ein C₁- bis C₄₋Alkylradikal oder
- wenn G³ und G⁴ in ortho-Stellung zueinander stehen, können sie gemeinsam eine verbrückende α,ω-Alkylendioxogruppe, wie beispielsweise einen Ethylendioxygruppe bilden.

Beispiele für die als Substituenten in den erfindungsgemäßen Verbindungen genannten, C₁- bis C₄-Alkylradikale sind die Gruppen Methyl, Ethyl, Propyl, Isopropyl und Butyl. Ethyl und Methyl sind bevorzugte Alkylradikale. Erfindungsgemäß bevorzugte C₁- bis C₄-Alkoxyradikale sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyalkylgruppe ist besonders bevorzugt. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (II) sind insbesondere die Aminogruppen, C₁- bis C₄₋Monoalkylaminogruppen, C₁- bis C₄-Dialkylaminogruppen, C₁- bis C₄₋Trialkylammoniumgruppen, C₁- bis C₄-Monohydroxyalkylaminogruppen, Imidazolinium und Ammonium.

Besonders bevorzugte p-Phenylendiamine der Formel (I) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-bis-(β-Hydroxyethyl)-p-phenylendiamin, 4-N,N-bis-(β-Hydroxyethyl)amino-2-methylanilin, 4-N,N-bis-(β-Hydroxyethyl)amino-2-chloranilin, 2-(β-Hydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,-β-hydroxyethyl)-p-phenylendiamin, N-(β,γ-Dihydroxypropyl)-p-phenylendiamin, N-(4'-Aminophenyl)-p-phenylendiamin, N-Phenylp-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (I) sind p-Phenylendiamin, p-Toluylendiamin, 2-(β-Hydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikemigen Entwicklerkomponenten, die in den Färbezusammensetzungen gemäß der Erfindung verwendet werden können, kann man insbesondere die Verbindungen nennen, die der folgenden Formel (II) entsprechen, sowie ihre physiologisch verträglichen Salze: 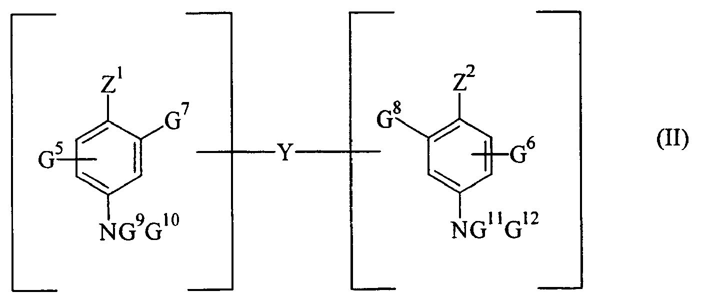
wobei:
- Z¹ und Z² stehen unabhängig voneinander für ein Hydroxyl- oder NH₂-Radikal, das gegebenenfalls durch ein C₁- bis C₄-Alkylradikal, durch ein C₁- bis C₄₋Hydroxyalkylradikal und/oder durch eine Verbrückung Y substituiert ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch ein oder mehrere Hydroxyl- oder C₁- bis C₈₋Alkoxyradikale substituiert sein kann,
- G⁵ und G⁶ stehen unabhängig voneinander für ein Wasserstoff- oder Halogenatom, ein C₁- bis C₄-Alkylradikal, ein C₁- bis C₄-Monohydroxyalkylradikal, ein C₂- bis C₄₋Polyhydroxyalkylradikal, ein C₁- bis C₄-Aminoalkylradikal oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder ein C₁- bis C₄₋Alkylradikal,
mit der Maßgabe, daß die Verbindungen der Formel (II) nur eine Verbrückung Y pro Molekül enthalten.

Die in Formel (II) verwendeten Substituenten sind erfindungsgemäß analog zu den obigen Ausführungen definiert.

Bevorzugte zweikernige Entwicklerkomponenten der Formel (II) sind insbesondere: N,N'-bis-(β-Hydroxyethyl)-N,N'-bis-(4'-Aminophenyl)-1,3-diamino-propanol, N,N'-bis-(β-Hydroxyethyl)-N,N'-bis-(4'-aminophenyl)-ethylendiamin, N,N'-bis-(4-Aminophenyl)-tetramethylendiamin, N,N'-bis-(β-Hydroxyethyl)-N,N'-bis-(4-aminophenyl)-tetramethylendiamin, N,N'-bis-(4-Methyl-aminophenyl)-tetramethylendiamin, N,N'-bis-(Ethyl)-N,N'-bis(4'-amino,3'-methylphenyl)-ethylendiamin, 1,8-bis-(2,5-Diaminophenoxy)-3,5-dioxaoktan, Bis-(2-hydroxy-5-aminophenyl)-methan, 1,4-Bis-(4-aminophenyl)-diaza-cycloheptan und 1,10-Bis-(2,5-diaminophenyl)-1,4,7,10-tetraoxadecan und ihre physiologisch verträglichen Salze.

Ganz besonders bevorzugte zweikernige Entwicklerkomponenten der Formel (II) sind N,N'-bis-(β-Hydroxyethyl)-N,N'-bis-(4'-aminophenyl)-1,3-diamino-propanol, Bis-(2-hydroxy-5-aminophenyl)-methan, N,N'-Bis-(4-aminophenyl)-1,4-diazacycloheptan und 1,10-Bis-(2,5-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (III) 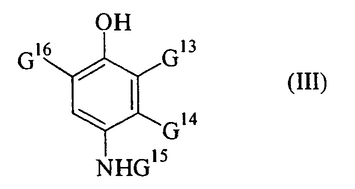 **wobei:**
- G¹³ steht für ein Wasserstoffatom, ein Halogenatom, ein C₁- bis C₄-Alkylradikal, ein C₁- bis C₄-Monohydroxyalkylradikal, ein (C₁- bis C₄)-Alkoxy-(C₁- bis C₄)-alkylradikal, ein C₁- bis C₄-Aminoalkylradikal, ein Hydroxy-(C₁- bis C₄)-alkylaminoradikal, ein C₁- bis C₄-Hydroxyalkoxyradikal, ein C₁- bis C₄₋Hydroxyalkyl-(C₁-bis C₄)-aminoalkylradikal oder ein (Di-C₁- bis C₄-Alkylamino)-(C₁- bis C₄)-alkylradikal, und
- G¹⁴ steht für ein Wasserstoff- oder Halogenatom, ein C₁- bis C₄-Alkylradikal, ein C₁₋bis C₄-Monohydroxyalkylradikal, ein C₂- bis C₄-Polyhydroxyalkylradikal, ein (C₁- bis C₄)-Alkoxy-(C₁- bis C₄)-alkylradikal, ein C₁- bis C₄-Aminoalkylradikal oder ein C₁- bis C₄-Cyanoalkylradikal,
- G¹⁵ steht für Wasserstoff, ein C₁- bis C₄-Alkylradikal, ein C₁- bis C₄₋Monohydroxyalkylradikal, ein C₂- bis C₄-Polyhydroxyalkylradikal, ein Phenylradikal oder ein Benzylradikal, und
- G¹⁶ steht für Wasserstoff oder ein Halogenatom.

Die in Formel (III) verwendeten Substituenten sind erfindungsgemäß analog zu den obigen Ausführungen definiert.

Bevorzugte p-Aminophenole der Formel (III) sind insbesondere p-Aminophenol, N-Methyl-p-Aminophenol, 4-Amino-3-methyl-phenol, 4-Amino-3-fluorphenol, 2-Hydroxymethylamino-4-amino-phenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-fluorophenol, 4-Amino-2-chlorphenol, 2,6-Dichlor-4-aminophenol, 4-Amino-2-((diethylamino)methyl)phenol sowie ihre physiologisch verträglichen Salze.

Ganz besonders bevorzugte Verbindungen der Formel (III) sind p-Aminophenol, 4-Amino-3-methylphenol, 4-Amino-2-aminomethylphenol und 4-Amino-2-((diethylamino)methyl)phenol.

Ferner kann die Entwicklerkomponente ausgewählt sein aus o-Aminophenol und seinen Derivaten, wie beispielsweise 2-Amino-4-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-Diamino-1-(β-hydroxyethyl)-pyrazol, 3,4-Diaminopyrazol, 4,5-Diamino-1-(4'-chlorobenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2'-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4(β-hydroxyethyl)amino-1-methylpyrazol.

Bevorzugte Pyrazol-Pyrimidin-Derivate sind insbesondere die Derivate des Pyrazol-[1,5-a]-pyrimidin der folgenden Formel (IV) und dessen tautomeren Formen, sofern ein tautomerisches Gleichgewicht besteht: 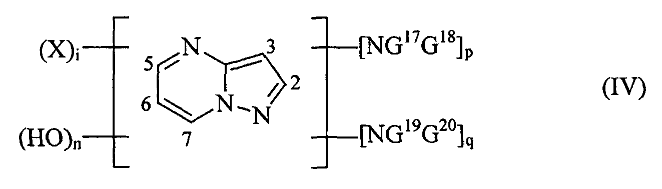 wobei:
- G¹⁷, G¹⁸, G¹⁹ und G²⁰ unabhängig voneinander stehen für ein Wasserstoffatom, ein C₁- bis C₄-Alkylradikal, ein Aryl-Radikal, ein C₁- bis C₄-Hydroxyalkylradikal, ein C₂- bis C₄-Polyhydroxyalkylradikal ein (C₁- bis C₄)-Alkoxy-(C₁- bis C₄)-alkylradikal, ein C₁- bis C₄-Aminoalkylradikal, das gegebenenfalls durch ein Acetyl-Ureid- oder Sulfonyl-Radikal geschützt sein kann, ein (C₁- bis C₄)-Alkylamino-(C₁- bis C₄)-alkylradikal, ein Di-[(C₁- bis C₄)-alkyl]-(C₁- bis C₄)-aminoalkylradikal, wobei die Dialkyl-Radikale gegebenenfalls einen Kohlenstoffzyklus oder einen Heterozyklus mit 5 oder 6 Kettengliedern bilden, ein C₁- bis C₄-Hydroxyalkyl- oder ein Di-(C₁- bis C₄)-[Hydroxyalkyl]-(C₁- bis C₄)-aminoalkylradikal,
- die X-Radikale stehen unabhängig voneinander für ein Wasserstoffatom, ein C₁- bis C₄-Alkylradikal, ein Aryl-Radikal, ein C₁- bis C₄-Hydroxyalkyladikal, ein C₂- bis C₄₋Polyhydroxyalkylradikal, ein C₁- bis C₄-Aminoalkylradikal, ein (C₁- bis C₄)-Alkylamino-(C₁- bis C₄)-alkylradikal, ein Di-[(C₁- bis C₄)alkyl]- (C₁- bis C₄)-aminoalkylradikal, wobei die Dialkyl-Radikale gegebenenfalls einen Kohlenstoffzyklus oder einen Heterozyklus mit 5 oder 6 Kettengliedern bilden, ein C₁- bis C₄-Hydroxyalkyl- oder ein Di-(C₁- bis C₄-hydroxyalkyl)-aminoalkylradikal, ein Aminoradikal, ein C₁- bis C₄-Alkyl- oder Di-(C₁- bis C₄-hydroxyalkyl)-aminoradikal, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, daß
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (IV) verwendeten Substituenten sind erfindungsgemäß analog zu den obigen Ausführungen definiert.

Wenn das Pyrazol-[1,5-a]-pyrimidin der obenstehenden Formel (IV) eine Hydroxygruppe an einer der Positionen 2, 5 oder 7 des Ringsystems enthält, besteht ein tautomeres Gleichgewicht, das zum Beispiel im folgenden Schema dargestellt wird:

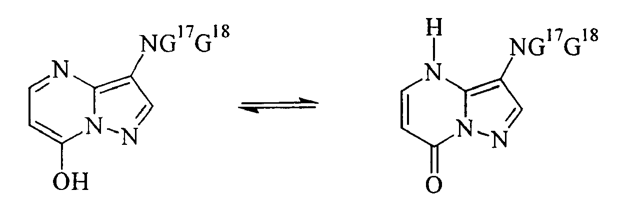

Unter den Pyrazol-[1,5-a]-pyrimidinen der obenstehenden Formel (IV) kann man insbesondere nennen:
- Pyrazol-[1,5-a]-pyrimidin-3,7-diamin;
- 2,5-Dimethyl pyrazol-[1,5-a]-pyrimidin-3,7-diamin;
- Pyrazol-(1,5-a]-pyrimidin-3,5-diamin;
- 2,7-Dimethyl pyrazol-[1,5-a]-pyrimidin-3,5-diamin;
- 3-Amino pyrazol-[1,5-a]-pyrimidin-7-ol;
- 3-Amino pyrazol-[1,5-a]-pyrimidin-5-ol;
- 2-(3-Amino pyrazol-[1,5-a]-pyrimidin-7-ylamino)-ethanol;
- 2-(7-Amino pyrazol-[1,5-a]-pyrimidin-3-ylamino)-ethanol;
- 2-[(3-Amino pyrazol-[1,5-a]-pyrimidin-7-yl)-(2-hydroxy-ethyl)-amino]-ethanol;
- 2-[(7-Amino pyrazol-[1,5-a]-pyrimidin-3-yl)-(2-hydroxy-ethyl)-amino]-ethanol;
- 5,6-Dimethyl pyrazol-[1,5-a]-pyrimidin-3,7-diamin;
- 2,6-Dimethyl pyrazol-[1,5-a]-pyrimidin-3,7-diamin;
- 2,5, N7, N7-Tetramethyl pyrazol-[1,5-a]-pyrimidin-3,7-diamin;
sowie ihre physiologisch verträglichen Salze und ihre tautomeren Formen, wenn ein tautomerisches Gleichgewicht vorhanden ist.

Die Pyrazol-[1,5-a]-pyrimidine der obenstehenden Formel (IV) können wie in der Literatur beschrieben durch Zyklisierung ausgehend von einem Aminopyrazol oder von Hydrazin hergestellt werden.

Besonders bevorzugte Entwicklerkomponenten sind p-Phenylendiamin, p-Toluylendiamin, p-Aminophenol, 1-(2'-Hydroxyethyl)-2,5-diaminobenzol, N,N-Bis-(2-hydroxy-ethyl)-p-phenylendiamin, 4-Amino-3-methylphenol, 4-Amino-2-((diethylamino)-methyl)-phenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin und 4.5-Diamino-1-(2`-hydroxyethyl)-pyrazol.

Zur Nuancierung der erzielbaren Farbtöne können im Rahmen des erfindungsgemäßen Verfahrens weiterhin noch eine oder mehrere Kupplerkomponenten eingesetzt werden. Kupplersubstanzen sind häufig aromatische oder heterocyclische Ringsysteme, die zwei reaktive Gruppen in meta-Stellung aufweisen. Als Kupplerkomponenten werden in der Regel m-Phenylendiaminderivate, Naphthole, Resorcin und Resorcinderivate, Pyrazolone und m-Aminophenolderivate verwendet.

Erfindungsgemäß bevorzugte Kupplerkomponenten sind
- m-Aminophenol und dessen Derivate wie beispielsweise 5-Amino-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-S-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Besonders bevorzugte Kuppler-Komponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Sulfate, einzusetzen.

Die Anwendungszubereitungen im Rahmen des erfindungsgemäßen Verfahrens enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Üblicherweise werden Entwicklerkomponenten und Kupplerkomponenten in etwa gleichen molaren Mengen zueinander eingesetzt. Wenn sich auch der äquimolare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten bevorzugt in einem Mol-Verhältnis von 1 : 0,5 bis 1 : 2 im Färbemittel enthalten sein können. Die Gesamtmenge an Oxidationsfarbstoffvorprodukten liegt in der Regel bei höchstens 20 Gew.-%, bezogen auf die gesamte Anwendungszubereitung.

Gemäß einer zweiten bevorzugten Ausführungsform des Gegenstandes der vorliegenden Erfindung kann das Farbstoffvorprodukt (A) ein Derivat des Indolins der Formel (Va) sein, 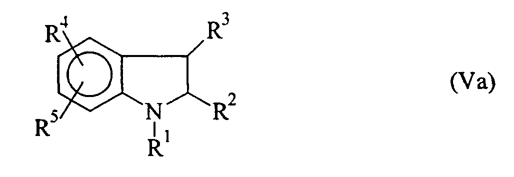 in der unabhängig voneinander R¹ steht für Wasserstoff, eine C₁- bis C₄-Alkylgruppe oder eine C₁- bis C₄-Hydroxy-alkylgruppe, R² steht für Wasserstoff oder eine -COOH-Gruppe, wobei die -COOH-Gruppe auch als Salz mit einem physiologisch verträglichen Kation vorliegen kann, R³ steht für Wasserstoff oder eine C₁- bis C₄-Alkylgruppe, R⁴ steht für Wasserstoff, eine Hydroxygruppe, eine Aminogruppe, eine C₁- bis C₄-Alkoxygruppe oder eine Gruppe -OCO-R⁶, in der R⁶ steht für eine C₁- bis C₄-Alkylgruppe, und R⁵ steht für eine der unter R⁴ genannten Gruppen, oder ein physiologisch verträgliches Salz dieser Verbindungen mit einer organischen oder anorganischen Säure.

In einer dritten bevorzugten Ausführungsform dieses Gegenstandes der vorliegenden Erfindung kann das Farbstoffvorprodukt (A) ein Derivat des Indols der Formel (Vb) sein, 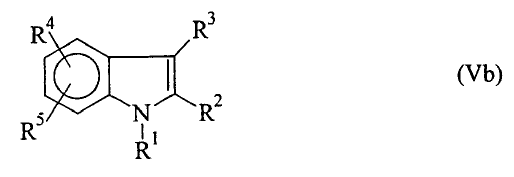 in der unabhängig voneinander R¹ steht für Wasserstoff, eine C₁- bis C₄-Alkylgruppe oder eine C₁- bis C₄-Hydroxy-alkylgruppe, R² steht für Wasserstoff oder eine -COOH-Gruppe, wobei die -COOH-Gruppe auch als Salz mit einem physiologisch verträglichen Kation vorliegen kann, R³ steht für Wasserstoff oder eine C₁- bis C₄-Alkylgruppe, R⁴ steht für Wasserstoff, eine Hydroxygruppe, eine Aminogruppe, eine C₁- bis C₄-Alkoxygruppe oder eine Gruppe -OCO-R⁶, in der R⁶ steht für eine C₁- bis C₄-Alkylgruppe, und R⁵ steht für eine der unter R⁴ genannten Gruppen, oder ein physiologisch verträgliches Salz dieser Verbindungen mit einer organischen oder anorganischen Säure.

Bevorzugte Stoffe der Formel (Va) sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin. Bevorzugte Stoffe der Formel (Vb) sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Ganz besonders bevorzugt sind 5,6-Dihydroxyindol sowie 5,6-Dihydroxyindolin.

In einer ersten bevorzugten Variante der oben beschriebenen Ausführungsformen wird die Zubereitung derart formuliert, daß sie als Farbstoffvorprodukte (A) nur Indol- und/oder Indolinderivate der Formeln (Va) und (Vb) enthalten und frei sind von üblichen Oxidationsfarbstoffvorprodukten vom Entwickler- bzw. Kupplertyp.

In einer zweiten bevorzugten Variante der oben beschriebenen Ausführungsformen kann die Zubereitung neben den Indol- und/oder Indolinderivaten der Formeln (Va) und (Vb) auch noch übliche Oxidationsfarbstoffvorprodukte vom Entwickler- bzw. Kupplertyp enthalten. In diesem Zusammenhang sei explizit auf die obigen Ausführungen verwiesen.

Es kann erfindungsgemäß besonders bevorzugt sein, die Indol- und/oder die Indolinderivate der Formeln (Va) und (Vb) in Kombination mit einer oder mehrere Kupplerkomponenten im Rahmen des erfindungsgemäßen Färbeverfahrens einzusetzen. Beispielhaft sei an dieser Stelle ausdrücklich auf die oben genannten Kupplerkomponenten verwiesen.

Weiterhin kann es erfindungsgemäß bevorzugt sein, die Indol- und/oder Indolinderivate der Formel (Va) und (Vb) in Kombination mit mindestens einer Aminosäure oder einem Oligopeptid im Rahmen des erfindungsgemäßen Färbeverfahrens einzusetzen. Es kann erfindungsgemäß weiterhin bevorzugt sein, wenn die Aminosäure eine α-Aminosäure ist. Ganz besonders bevorzugte α-Aminosäuren sind Arginin, Ornithin, Lysin und Histidin.

In einer bevorzugten Ausführungsform enthält die Zubereitung zur weiteren Modifizierung der Farbnuancen neben den Farbstoffvorprodukten zusätzlich übliche direktziehende Farbstoffe. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, HC Orange 1, Disperse Orange 3, HC Red 1, HC Red 3, HC Red 13, HC Red BN, Basic Red 76, HC Blue 2, HC Blue 12, Disperse Blue 3, Basic Blue 7, Basic Blue 26, Basic Blue 99, HC Violet 1, Disperse Violet 1, Disperse Violet 4, Basic Violet 2, Basic Violet 14, Acid Violet 43, Disperse Black 9, Acid Black 52, Basic Brown 16 und Basic Brown 17 bekannten Verbindungen sowie 1,4-Bis-(β-hydroxyethyl)-amino-2-nitrobenzol. 3-Nitro-4-(β-hydroxyethyl)-aminophenol, 4-Amino-2-nitrodiphenylamin-2'-carbonsäure, 6-Nitro-1,2,3,4-tetrahydrochinoxalin, 2-Hydroxy-1,4-naphthochinon, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol.

Die direktziehenden Farbstoffe werden bevorzugt in einer Menge von 0,01 bis 20 Gew.-%, bezogen auf die Anwendungszubereitung, eingesetzt.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die Farbstoffvorprodukte oder die direktziehenden Farbstoffe jeweils einheitliche Verbindungen darstellen. Vielmehr können in den erfindungsgemäßen Färbeverfahren, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Färbeverfahren einsetzbaren Farbstoffe wird weiterhin ausdrücklich auf die Monographie Ch. Zviak, The Science of Hair Care, Kapitel 7 (Seiten 248-250; direktziehende Farbstoffe), sowie Kapitel 8, Seiten 264-267; Oxidationsfarbstoffvorprodukte), erschienen als Band 7 der Reihe "Dermatology" (Hrg.: Ch. Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Färbungen von besonderer Farbtiefe können erreicht werden, wenn die Anwendungszubereitung neben den Farbstoffen und/oder Farbstoffvorprodukten zusätzlich noch ein Öl des Wiesenschaumkrauts (INCI-Bezeichnung: Meadowfoam Seed Oil) enthält.

Im Rahmen des erfindungsgemäßen Verfahrens kann die oxidative Entwicklung der Färbung grundsätzlich mit Luftsauerstoff erfolgen. Bevorzugt wird jedoch ein chemisches Oxidationsmittel eingesetzt, besonders dann, wenn neben der Färbung ein Aufhelleffekt am menschlichen Haar gewünscht ist. Als Oxidationsmittel kommen insbesondere Wasserstoffperoxid oder dessen Anlagerungsprodukte an Harnstoff, Melamin oder Natriumborat in Frage. In einer bevorzugten Ausführungsform des erfindungsgemäßen Verfahrens wird daher zusätzlich eine Zubereitung zugegeben, die mindestens ein Oxidationsmittel (C) enthält.

Insbesondere bei schwer färbbarem Haar kann die Zubereitung mit den Farbstoffvorprodukten und den Rutheniumverbindungen ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert.

Es kann erfindungsgemäß bevorzugt sein, daß die Komponenten (A), (B) und (C) bis unmittelbar vor der Anwendung separat konfektioniert sind. Erfindungsgemäß umfaßt sollen aber auch alle Ausführungsformen sein, bei denen zwei dieser Komponenten gemeinsam konfektioniert sind und die dritte Komponente erst unmittelbar vor der Anwendung hinzugegeben wird. Bei der Herstellung der Anwendungszubereitung aus den einzelnen Zubereitungen mit den Komponenten (A), (B) und (C) sind prinzipiell alle Variationen denkbar. So kann beispielsweise zunächst eine Mischung der Zubereitungen der Komponenten (A) und (B) hergestellt werden, die dann anschließend mit der Zusammensetzung (C) vermischt wird. Weiterhin können aber auch zunächst die Zubereitungen der Komponenten (A) und (C) vermischt werden und abschließend (B) hinzugefügt werden. Auch eine Mischung der Zubereitungen der Komponenten (B) und (C) bei anschließender Zugabe von (A) soll erfindungsgemäß umfaßt sein.

Die Rutheniumverbindungen können erfindungsgemäß sowohl in einer gemeinsamen Zubereitung mit den Farbstoffvorprodukten als auch separat konfektioniert sein.

In einer bevorzugten Ausführungsform der erfindungsgemäßen Lehre werden die separat konfektionierten Übergangsmetallkomplexe in einem geeigneten Lösemittel, beispielsweise in Wasser, Ethanol oder Aceton, gelöst und unmittelbar vor dem Färben der Haare mit der Oxidationsmittelzubereitung verrührt. Diese Zubereitung wird anschließend mit einer Zubereitung, enthaltend die Farbstoffvorprodukte (A), vermischt.

Weiterhin ist es möglich, die Oxidation mit Hilfe von Enzymen zu beschleunigen, wobei die Enzyme sowohl zur Erzeugung von oxidierenden Per-Verbindungen eingesetzt werden als auch zur Verstärkung der Wirkung einer geringen Menge vorhandener Oxidationsmittel. So können die Enzyme (Enzymklasse 1: Oxidoreduktasen) Elektronen aus geeigneten Entwicklerkomponenten (Reduktionsmittel) auf Luftsauerstoff übertragen. Bevorzugt sind dabei Oxidasen wie Tyrosinase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Das bei jeder der Varianten entstehende gebrauchsfertige Haarfärbepräparat sollte bevorzugt einen pH-Wert im Bereich von 6 bis 10 aufweisen. Besonders bevorzugt ist ein pH-Wert von 6,5 bis 8. Die Anwendungstemperaturen können in einem Bereich zwischen 15 und 40°C liegen. Die Anwendungszubereitung kann bevorzugterweise nach einer Einwirkzeit von 3 bis 120 Minuten ausgespült werden. Dieses Ausspülen kann mit reinem Wasser erfolgen. Einwirkzeiten von 15 bis 30 Minuten haben sich in den meisten Fällen als ausreichend erwiesen. Das Nachwaschen mit einem Shampoo entfällt, wenn ein stark tensidhaltiger Träger, z.B. ein Färbeshampoo, verwendet wurde.

Ein zweiter Gegenstand der vorliegenden Erfindung sind Mittel zum Färben keratinischer Fasern, insbesondere menschlicher Haare, enthaltend (A) mindestens ein Farbstoffvorprodukt und (B) mindestens ein einfaches Rutheniumsalz und/oder einen Rutheniumkomplex mit acyclischen Liganden, mit der Maßgabe, daß die Rutheniumsalze und/oder Rutheniumkomplexe nicht in Hohlräume von Käfigverbindungen eingeschlossen sind.

Zur Herstellung der erfindungsgemäßen Färbemittel werden die Farbstoffvorprodukte in einen geeigneten wäßrigen, alkoholischen oder wäßrig-alkoholischen Träger eingearbeitet. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Unter wäßrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen.

Die erfindungsgemäßen Färbemittel können weiterhin alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Anionische Tenside können dabei ganz besonders bevorzugt sein.

Als anionische Tenside eignen sich in erfindungsgemäßen Färbemittel alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ether-, Amid- und Hydroxylgruppen sowie in der Regel auch Estergruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare und verzweigte Fettsäuren mit 8 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(-CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylen glykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungs produkte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure. Nichtionische Tenside enthalten als hydrophile Gruppe z. B. eine Polyolgruppe, eine Polyalkylenglykolethergruppe oder eine Kombination aus Polyol- und Polyglykol-ethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂- bis C₂₂-Fettsäuremono- und -diester von Anlagerungsprodukten von 1 bis 30 Mol Ethylenoxid an Glycerin,
- C₈- bis C₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester,
- Anlagerungsprodukte von Ethylenoxid an Fettsäurealkanolamide.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethyl-ammoniumglycinate, beispielsweise das Kokosacylaminopropyl-dimethyl-ammonium-glycinat, und 2-Alkyl-3-carboxymethyl-3-hydroxyethyl-imidazoline mit jeweils 8 bis 18 C-Atomen in der Alkyl- oder Acylgruppe sowie das Kokosacyl-aminoethyl-hydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈- bis C₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂-bis C₁₈-Acylsarcosin.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldi-methylammoniumchloride und Trialkylmethylammoniumchloride, z. B. Cetyltrimethyl-ammoniumchlorid, Stearyltrimethylammoniumchlorid, Distearyldimethylammonium-chlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224® (Hersteller: Dow Corning; ein stabilisiertes Trimethylsilylamodimethicon), Dow Corning 929® Emulsion (enthaltend ein hydroxyl-amino-modifiziertes Silicon, das auch als Amodimethicone bezeichnet wird), SM-2059® (Hersteller: General Electric), SLM-55067® (Hersteller: Wacker) sowie Abil®-Quat 3270® und 3272® (Hersteller: Th. Goldschmidt; diquatemäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyl-dialkoyloxyalkyl-ammonium-methosulfate sowie die unter dem Warenzeichen Dehyquart® vertriebenen Produkte.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Je nach Art des Mittels und des Tensidtyps sind die Tenside in den erfindungsgemäßen Mitteln üblicherweise in Mengen von insgesamt 0,5 bis 30 Gew.-%, bezogen auf das gesamte Mittel, enthalten.

Weiterhin können die erfindungsgemäßen Haarbehandlungsmittel bevorzugt noch einen konditionierenden Wirkstoff, ausgewählt aus der Gruppe, die von kationischen Tensiden, kationischen Polymeren. Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat®100 (Poly(dimethyldiallylammoniumchlorid)), Merquat®550 (Dimethyldiallylammoniumchlorid-Acrylamid-Copolymer) und Merquat® 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat®734 und Gafquat®755 im Handel erhältlich.
- Vinylpyrrolidon-Methoimidazoliniumchlorid-Copolymere, wie sie unter der Bezeichnung Luviquat® angeboten werden.
- quaternierter Polyvinylalkohol
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquatemium-10 und Polyquatemium-22.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190®, DC 200®, DC 344®, DC 345® und DC 1401® vertriebenen Produkte sowie die Handelsprodukte Q2-7224® (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059® (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Schließlich enthalten die erfindungsgemäßen Färbemittel bevorzugt noch einen Fettstoff.

Bevorzugte Fettstoffe sind lineare und verzweigte, gesättigte und ungesättigte Fettalkohole oder natürliche Fettalkoholgemisch mit 8 bis 22 Kohlenstoffatomen in der Alkylkette wie beispielsweise Decanol, Octanol, Octenol, Dodecenol, Decenol, Octadienol, Dodecadienol, Decadienol, Oleylalkohol, Erucaalkohol, Ricinolalkohol, Stearylalkohol. Isostearylalkohol, Palmitylalkohol, Laurylalkohol, Myristylalkohol, Arachidylalkohol. Caprylalkohol, Caprinalkohol, Linoleylalkohol, Linolenylalkohol und Behenylalkohol, sowie deren Guerbetalkohole sowie Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden und somit ein Gemisch von unterschiedlichen Fettalkoholen darstellen. Die Fettalkohole werden üblicherweise in Mengen von 0,01 bis 15 Gew.-%, bevorzugt von 0,1 bis 10 Gew.-% und besonders bevorzugt von 0,3 bis 6 Gew.-%, bezogen auf die gesamte Zubereitung, eingesetzt.

Ebenfalls als Fettstoffe eingesetzt werden können Monoester der Fettsäuren mit Alkoholen mit 6 bis 24 C-Atomen sowie Triglyceride natürlichen Ursprungs.

Weitere Wirk-, Hilfs- und Zusatzstoffe sind beispielsweise
- nichtionische Polymere wie beispielsweise Vinylpyrrolidon/Vinylacrylat-Copolymere. Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylamid/Methylmethacry-lat/tert.Butylaminoethylmethacrylat/2-Hydroxypropylmethacrylat-Copolymere,
- anionische Polymere wie beispielsweise Polyacrylsäuren, vemetzte Polyacrylsäuren, Vinylacetat/Crotonsäure-Copolymere, Vinylpyrrolidon/Vinylacrylat-Copolymere, Vi-nylacetat/Butylmaleat/Isobornylacrylat-Copolymere, Methylvinylether/Maleinsäure-anhydrid-Copolymere und Acrylsäure/Ethylacrylat/N-tert.-Butylacrylamid-Terpoly-mere,
- symmetrische und unsymmetrische, lineare und verzweigte Dialkylether mit insgesamt zwischen 12 bis 36 C-Atomen, insbesondere 12 bis 24 C-Atomen, beispielsweise Di-n-octylether, Di-n-decylether, Di-n-nonylether, Di-n-undecylether und Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert-butylether, Di-iso-pentylether, Di-3-ethyldecylether, tert.-Butyl-n-octylether, isoPentyl-n-octylether und 2-Methyl-pentyl-n-octylether,
- Antischuppenwirkstoffe wie Piroctone Olamine, Zink Omadine und Climbazol,
- tierische und pflanzliche Proteinhydrolysate, insbesondere Elastin-, Kollagen-, Keratin-, Milchciweiß-, Sojaprotein-, Mandelprotein- und Weizenproteinhydrolysate, sowie deren Fettsäurekondensationsprodukte und quaternierte Derivate,
- Vitamine und Vitaminvorstufen wie Panthenol, dessen Derivate und Biotin,
- Pflanzen- und Honigextrakte, wie insbesondere Extrakte aus Eichenrinden, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe. Hauhechel, Meristem, Ginseng und Ingwerwurzel,
- Weitere Wirkstoffe wie Ceramide, Allantoin, Pyrrolidoncarbonsäuren, und Bisabolol.
- Lichtschutzmittel.
- Entschäumer wie Silikone,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose. Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose. Maleinsäure und Milchsäure,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Alkalisierungsmittel wie beispielsweise Ammoniak, Monoethanolamin, 2-Amino-2-methylpropanol und 2-Amino-2-methyl-propandiol-1,3
- weitere Substanzen zur Einstellung des pH-Wertes,
- Cholesterin.
- Quell- und Penetrationsstoffe wie Glycerin, Propylenglykolmonoethylether, Carbonate, Hydrogencarbonate. Guanidine, Harnstoffe sowie primäre, sekundäre und tertiäre Phosphate,
- Konsistenzgeber wie Zuckerester, Polyolester oder Polyolalkylether,
- Strukturanten wie Maleinsäure, Mono-, Di- und Oligosaccharide,
- Fette und Wachse, wie Walrat, Bienenwachs, Montanwachs und Paraffine,
- Fettsäurealkanolamide,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Komplexbildner wie EDTA, NTA, β-Alanindiessigsäure und Phosphonsäuren,
- Treibmittel wie Propan-Butan-Gemische, N₂O, Dimethylether, CO₂, N₂ und Luft

Bezüglich weiterer Bestandteile sowie Mengenbereiche für die einzelnen Inhaltsstoffe wird auf die dem Fachmann bekannten Handbücher, z.B. K. Schrader, Grundlagen und Rezepturen der Kosmetika, 2. Auflage, Hüthig Buch Verlag, Heidelberg, 1989, verwiesen.

Durch die erfindungsgemäße Verwendung der Rutheniumverbindungen ist es möglich
- die üblicherweise nur unter alkalischen Bedingungen erhaltenen Färbeleistungen auch im neutralen pH-Bereich zu erzielen oder
- eine schonendere Färbebehandlung dadurch zu erzielen, daß im alkalischen Bereich die angestrebte Färbeleistung bereits mit deutlich, d.h. bis zu 75 %, niedrigeren Konzentrationen an Oxidationsmittel erreicht wird. So kann beispielsweise die Wasserstoffperoxidkonzentration in einer Anwendungszubereitung von ca. 3 Gew.-% auf ca. 1,0 Gew.-% gesenkt werden.

### Beispiele

### I) Prinzipielle Herstellungsweise der Färbecreme und Anwendungsbeispiel

Es wurde eine Färbecreme folgender Zusammensetzung hergestellt (alle Angaben sind, soweit nicht anders vermerkt, in g):

| **Teilmischung A** | |
|---|---|
| Hydrenol® D¹ | 8,50g |
| Lorol® techn.² | 2,00g |
| Eumulgin® B2³ | 0,75g |
| Texapon® NSO⁴ | 20,00g |
| Dehyton® K⁵ | 12,50g |
| Wasser | 30,00g |

| | |
|---|---|
| ¹ C₁₆₋₁₈-Fettalkohol (INCI-Bezeichnung: Cetearyl alcohol) (HENKEL) | |
| ² C₁₂₋₁₈-Fettalkohol (INCI-Bezeichnung: Coconut alcohol) (HENKEL) | |
| ³ Cetylstearylalkohol mit ca. 20 EO-Einheiten (INCI-Bezeichnung: Ceteareth-20) (HENKEL) | |
| ⁴ Laurylethersulfat, Natriumsalz (ca. 27,5% Aktivsubstanz; INCI-Bezeichnung: Sodium Laureth Sulfate) (HENKEL) | |
| ⁵ N,N-Dimethyl-N-(C₈₋₁₈-kokosamidopropyl)ammoniumacetobetain (ca. 30% Aktivsubstanz; INCI-Bczeichnung: Aqua (Water), Cocamidopropyl Betaine) (HENKEL) | |

Die Substanzen Hydrenol® D, Lorol® und Eumulgin® B2 wurden bei 80°C aufgeschmolzen, mit dem 80°C heißem Wasser, enthaltend Texapon® NSO und Dehyton®K vermischt und unter starkem Rühren emulgiert. Danach wurde die Emulsion unter kontinuierlichem Rühren abgekühlt.

| **Teilmischung B** | |
|---|---|
| Natriumsulfit | 1,00g |
| Ammoniumsulfat | 1,00g |
| 5-Amino-2-methylphenol | 0,003mol |
| 4-Amino-2-aminomethylphenol-hydrochlorid | 0,003mol |
| Ammoniak (25%ige Lösung) | ad pH 8,7 |
| Wasser | 10,00g |

Die Farbstoffvorprodukte wurden in dem 50°C heißem Wasser unter Zugabe von Natriumsulfit. Ammoniumsulfat und Ammoniak gelöst.

Die Farbstoffvorproduktlösung (Teilmischung B) wurde zur Emulsion (Teilmischung A) gegeben, mit einer Ammoniaklösung auf den gewünschten pH-Wert eingestellt und mit Wasser auf 100 g aufgefüllt. Es wurde bis zum Erreichen der Raumtemperatur weitergerührt. Die Färbecreme wies einen pH-Wert von 8,7 auf.

Als Entwickler wurden 10 ml einer 3 Gew.-%igen wäßrigen Wasserstoffperoxidlösung verwendet. Die Rutheniumverbindung wurde in einigen Tropfen Wasser vorgelöst und zu der Entwicklerlösung gegeben. Sodann wurden Entwicklerlösung und 12,5g Färbecreme vereinigt; die anwendungsbereite Färbemischung hatte jeweils einen pH-Wert von 7,0 - 7,5. Anschließend wurde die Anwendungszubereitung auf eine Haarsträhne der Sorte "Kerling Naturweiß" (11g Anwendungszubereitung pro 1g Haar), gegeben, dort 30 Minuten belassen und dann mit Wasser ausgespült.

Es wurden Anwendungszubereitungen mit einer Konzentration von 222ppm Ruthenium sowie 111ppm Ruthenium, jeweils bezogen auf die gesamte Anwendungszubereitung, hergestellt.

| **Metallsatz** | **Ru-Konzentration** | **Bewertung** |
|---|---|---|
| % | % | - |
| RuCl₃ x 3 H₂O | 111 ppm | ++ |
| RuCl₃ x 3 H₂O | 222ppm | +++ |
| [Ru(NH₃)₆]Cl₃ | 111ppm | +++ |

| | | |
|---|---|---|
| Bewertungsmaßstab - keine Färbung | | |
| + leichte Färbung | | |
| ++ gute Färbung, leicht schwächer als Positivstandard | | |
| +++ Vollaustarbung, vom Positivstandard nicht zu unterscheiden | | |

Als Nullwert diente das Färbeergenbis eines Versuchs ohne Katalysatorzusatz bei pH 7,0, bei dem keine Färbung beobachtet werden konnte. Als Positivstandard diente eine Strähne, die mit der oben genannten Anwendungszubereitung (12,5 g Färbecreme mit 10ml einer 3%igen Wasserstoffperoxidlösung vermischt) bei einem pH-Wert von 9,5 ausgefärbt wurde.

### II) Detaillierte Untersuchungen zur Färbung im Kupferbereich

Um den Gegenstand der Erfindung noch stärker zu verdeutlichen, wurden einige Untersuchungen zur Nuancierung im Kupferbereich durchgeführt.

Ziel der vorliegenden Erfindung war es, die gewünschte Nuancierung durch den Zusatz von RuCl₃, x 3H₂O und die Absenkung des pH-Wertes der Anwendungszubereitung auf einen pH-Wert im neutralen Bereich (im Vergleich zur Standardausfärbung im alkalischen Bereich) nicht zu verschieben.

Die Herstellung der Färbecreme erfolgte analog der unter Punkt I) beschriebenen Methode, wobei die in der Teilmischung B angegebenen Farbstoffvorprodukte durch die in der Tabelle 1 angegebenen Farbstoffvorprodukte ersetzt wurden. Für die anschließende Ausfärbung wurden 6.25g der entsprechenden Färbecreme mit 2,5g einer 3 Gew.-% Wasserstoffperoxid enthaltenden, wässerigen Entwicklerlösung sowie gegebenenfalls 1,25g einer 0.1 Gew.%igen wässerigen Lösung von RuCl₃ x 3 H₂O gemischt. Der pH-Wert der Anwendungszubereitung wurde anschließend jeweils auf den gewünschten Wert eingestellt (Siehe Tabelle 2) und diese dann auf naturweiße Haarsträhnen von 0,5 g Gewicht aufgetragen. Die Haarsträhnen wurden 30 Minuten bei 32°C ausgefärbt und anschließend sorgfältig abgespült und getrocknet.

**Tabelle 1**

| **Farbstoffvorprodukt** | **Rezeptur Nr. (Mengenangaben in Gew.%)** | | |
|---|---|---|---|
| | **1** | **2** | **3** |
| p-Toluylendiamin-sulfat | 0,132 | 0,783 | 0,088 |
| 4-Amino-2-aminomethylphenol-hydrochlorid | 0,248 | 0,470 | -- |
| 4-Amino-3-methylphenol | 0,145 | 0,018 | -- |
| Resorcin | 0,214 | 0,396 | 0,15 |
| 5-Amino-2-methylphenol | 0,113 | 0,022 | 0,023 |
| 3-Amino-2-chlor-6-methylphenol | -- | 0,045 | -- |
| 1,3-Bis-(2',4'-diaminophenoxy)-propan-tetrahydrochlorid | -- | -- | 0,046 |
| 2,4,5,6-Tetraaminopyrimidin-sulfat | -- | -- | 1,924 |
| 2,7-Dihydroxnaphihalin | -- | -- | 0,423 |
| 2-Methylresorcin | -- | -- | 0,337 |
| 2,6-Dihydroxy-3,4-dimethylpyridin | -- | -- | 0,035 |

Die nachfolgende Tabelle 2 gibt die erhaltenen Färbeergebnisse wieder:

**Tabelle 2:**

| | **pH-Wert der Anwendungszubereitung** | **RuCl**_{**3**} **x 3H**_{**2**}**O-Lösung** | **Nuance** |
|---|---|---|---|
| 1a | 9,4 | -- | Kupfergold, Farbtiefe Mittelblond |
| 1b | 7,6 | -- | bräunliches Kupfer, Farbtiefe hellblond bis mittelblond |
| 1c | 7,6 | + | Kupfergold, Farbtiefe hellblond bis mittelblond |
| 2a | 9,4 | -- | Goldbraun, Farbtiefe hellbraun |
| 2b | 7,6 | -- | Violettbraun, Farbtiefe hellbraun |
| 2c | 7,6 | + | Goldbraun, Farbtiefe dunkelblond bis hellbraun |
| 3a | 9,4 | -- | Kirschrot, Farbtiefe hellbraun bis dunkelbraun |
| 3b | 7,6 | -- | Gold, Farbtiefe dunkelblond |
| 3c | 7,6 | + | Kirschrot, Farbtiefe dunkelblond |

Den Färbeergebnissen kann entnommen werden, daß es bei einer Absenkung des pH-Wertes der Anwendungszubereitung von 9,4 auf 7,6 ohne den Einsatz des erfindungsgemäßen Katalysators zu einer deutlichen Farbverschiebung unter Verminderung des Rotanleils kommen kann (jeweils Vergleich der Versuche a und b). Bei Zusatz des Katalysators (jeweils Versuch c) kann jedoch die Farbnuancierung, verbunden mit einer leichten Aufhellung, beibehalten werden.

## Patentansprüche

1. Verfahren zum Färben keratinischer Fasern, insbesondere menschlicher Haare, bei dem eine Zubereitung, enthaltend
(A) mindestens ein Farbstoffvorprodukt und
(B) mindestens ein einfaches Rutheniumsalz und/oder einen Rutheniumkomplex mit acyclischen Liganden,
auf die Fasern aufgetragen wird und nach einer Einwirkzeit wieder abgespült wird, mit der Maßgabe, daß die Rutheniumsalze und/oder Rutheniumkomplexe nicht in Hohlräumen von Käfigverbindungen eingeschlossen sind.

2. Verfahren nach Anspruch 1, **dadurch gekennzeichnet, daß** die Zubereitung als Komponente (B) eine Verbindung des Rutheniums in der Oxidationsstufe +3 enthält.

3. Verfahren nach Anspruch 1 oder 2, **dadurch gekennzeichnet, daß** die Zubereitung als Komponente (B) eine Verbindung aus mindestens einem Rutheniumkation und mindestens einem Chloridanion enthält.

4. Verfahren nach einem der Ansprüche 1 bis 3, **dadurch gekennzeichnet, daß** der Rutheniumkomplex (B) mindestens einen neutralen, einzähnigen acyclischen Liganden enthält.

5. Verfahren nach Anspruch 4. **dadurch gekennzeichnet, daß** der neutrale, einzähnige Ligand ausgewählt ist aus Wasser und/oder Ammoniak.

6. Verfahren nach einem der Ansprüche 1 bis 5, **dadurch gekennzeichnet, daß** die Zubereitung als Komponente (B) RuCl₃ x 3 H₂O und / oder [Ru(NH₃)₆]Cl₃ enthält.

7. Verfahren nach einem der Ansprüche 1 bis 6, **dadurch gekennzeichnet, daß** die Zubereitung mindestens ein Farbstoffvorprodukt (A) vom Typ der Entwickler enthält.

8. Verfahren nach einem der Ansprüche 1 bis 7, **dadurch gekennzeichnet, daß** die Zubereitung als Farbstoffvorprodukt (A) mindestens ein Indol- und/oder Indolinderivat enthält.

9. Verfahren nach einem der Ansprüche 1 bis 8, **dadurch gekennzeichnet, daß** die Anwendunngszubereitung einen pH-Wert von 6,5 bis 8 aufweist.

10. Verfahren nach einem der Ansprüche 1 bis 9, **dadurch gekennzeichnet, daß** zusätzlich eine Zubereitung zugegeben wird, die mindestens ein Oxidationsmittel (C) enthält.

11. Mittel zum Färben keratinischer Fasern, insbesondere menschlicher Haare, enthaltend
(A) mindestens ein Farbstoffvorprodukt und
(B) mindestenes ein einfaches Rutheniumsalz und/oder einen Rutheniumkomplex mit acyclischen Liganden,
mit der Maßgabe, daß die Rutheniumsalze und/oder Rutheniumkomplexe nicht in Hohlräumen von Käfigverbindungen eingeschlossen sind.

## Claims

1. A process for colouring keratin fibres, more particularly human hair, in which a preparation containing
(A) at least one dye precursor and
(B) at least one simple ruthenium salt and/or a ruthenium complex with acyclic ligands
is applied to the fibres and, after a contact time, is rinsed off again, with the proviso that the ruthenium salts and/or ruthenium complexes are not enclosed in interstices of clathrate compounds.

2. A process as claimed in claim 1, **characterized in that** the preparation contains a compound of ruthenium in the oxidation stage +3 as component (B).

3. A process as claimed in claim 1 or 2, **characterized in that** the preparation contains a compound of at least one ruthenium cation and at least one chloride anion as component (B).

4. A process as claimed in any of claims 1 to 3, **characterized in that** the ruthenium complex (B) contains at least one neutral, monodentate acyclic ligand.

5. A process as claimed in claim 4, **characterized in that** the neutral monodentate ligand is selected from water and/or ammonia.

6. A process as claimed in any of claims 1 to 5, **characterized in that** the preparation contains RuCl₃ x 3 H₂O and/or [Ru(NH₃)₆]Cl₃ as component (B).

7. A preparation as claimed in any of claims 1 to 6, **characterized in that** the preparation contains at least one dye precursor (A) of the primary intermediate type.

8. A process as claimed in any of claims 1 to 7, **characterized in that** the preparation contains at least one indole and/or indoline derivative as the dye precursor (A).

9. A process as claimed in any of claims 1 to 8, **characterized in that** the preparation applied has a pH of 6.5 to 8.

10. A process as claimed in any of claims 1 to 9, **characterized in that** a preparation containing at least one oxidizing agent (C) is also added.

11. A preparation for colouring keratin fibres, more particularly human hair, containing
(A) at least one dye precursor and
(B) at least one simple ruthenium salt and/or a ruthenium complex with acyclic ligands,
with the proviso that the ruthenium salts and/or ruthenium complexes are not enclosed in interstices of clathrate compounds.

## Revendications

1. Procédé de coloration de fibres kératiniques, en particulier de cheveux humains, dans lequel on dépose sur les fibres une préparation contenant
(A) au moins un précurseur de colorant et
(B) au moins un sel de ruthénium simple et/ou un complexe de ruthénium avec des ligands acycliques,
et l'on rince à nouveau après un temps d'action, sous réserve que les sels de ruthénium et/ou les complexes de ruthénium ne sont pas inclus dans les espaces vides de produits d'inclusion en cage.

2. Procédé selon la revendication 1, **caractérisé en ce que** la préparation contient en tant que composant (B) un composé de ruthénium de degré d'oxydation +3.

3. Procédé selon la revendication 1 ou 2, **caractérisé en ce que** la préparation contient en tant que composant (B) un composé constitué d'au moins un cation ruthénium et d'au moins un anion chlorure.

4. Procédé selon l'une des revendications 1 à 3, **caractérisé en ce que** le complexe de ruthénium (B) contient au moins un ligand acyclique monodenté neutre.

5. Procédé selon la revendication 4, **caractérisé en ce que** le ligand monodenté neutre est choisi entre l'eau et/ou l'ammoniac.

6. Procédé selon l'une des revendications 1 à 5, **caractérisé en ce que** la préparation contient comme composant (B) RuCl₃ x 3 H₂O et/ou [Ru(NH₃)₆]Cl₃.

7. Procédé selon l'une des revendications 1 à 6, **caractérisé en ce que** la préparation contient au moins un précurseur de colorant (A) du type des agents révélateurs.

8. Procédé selon l'une des revendications 1 à 7, **caractérisé en ce que** la préparation contient comme précurseur de colorant (A) au moins un dérivé d'indole et/ou d'indoline.

9. Procédé selon l'une des revendications 1 à 8, **caractérisé en ce que** la préparation d'application présente un pH de 6,5 à 8.

10. Procédé selon l'une des revendications 1 à 9, **caractérisé en ce qu'**on ajoute de façon additionnelle une préparation qui contient au moins un oxydant (C).

11. Produit de coloration des fibres kératiniques, en particulier des cheveux humains, contenant
(A) au moins un précurseur de colorant et
(B) au moins un sel de ruthénium simple et/ou un complexe de ruthénium avec des ligands acycliques,
sous réserve que les sels de ruthénium et/ou les complexes de ruthénium ne sont pas inclus dans les espaces vides de produits d'inclusion en cage.
